# EUROPÄISCHE PATENTSCHRIFT

(11) **EP 3 180 382 B1**
(45) Veröffentlichungstag und Bekanntmachung des Hinweises auf die Patenterteilung: **10.10.2018**
(21) Anmeldenummer: 15750381.4
(22) Anmeldetag: 07.08.2015
(51) Int. Cl.: C08G 59/50, C07C 209/22, C07C 211/18, C07C 211/27

(54) **AMIN FÜR EMISSIONSARME EPOXIDHARZ-ZUSAMMENSETZUNGEN**
AMINE FOR LOW-EMISSION EPOXY RESIN COMPOSITIONS
AMINE POUR DES COMPOSITIONS DE RÉSINE ÉPOXY À FAIBLE ÉMISSION

(30) Priorität: 13.08.2014 EP 14180870
(43) Veröffentlichungstag der Anmeldung: 21.06.2017
(73) Patentinhaber: Sika Technology AG, 6340 Baar (CH)
(72) Erfinder: KASEMI, Edis, CH-8046 Zürich (CH); KRAMER, Andreas, CH-8006 Zürich (CH); STADELMANN, Ursula, CH-8046 Zürich (CH); BURCKHARDT, Urs, CH-8049 Zürich (CH)
(74) Vertreter: Sika Patent Attorneys
(86) Internationale Anmeldenummer: PCT/EP2015/068300
(87) Internationale Veröffentlichungsnummer: WO 2016/023837

(56) Entgegenhaltungen:
- EP-A1- 2 465 843

## Beschreibung

### Technisches Gebiet

Die Erfindung betrifft das Gebiet der Amine, Härter für Epoxidharze, Epoxidharz-Zusammensetzungen, sowie deren Verwendung, insbesondere als Beschichtung, Belag oder Anstrich.

### Stand der Technik

Für Beschichtungszwecke geeignete Epoxidharz-Zusammensetzungen sollen eine möglichst niedrige Viskosität aufweisen, damit sie bei Umgebungstemperatur gut verarbeitbar sind. Weiterhin sollen sie möglichst schnell und störungsfrei aushärten, auch bei feucht-kalten Bedingungen, und dabei eine ebenmässige Oberfläche ohne Trübungen, Flecken oder Krater ausbilden. Schliesslich soll eine ausgehärtete Beschichtung eine hohe Härte bei geringer Sprödigkeit besitzen, um mechanischer Beanspruchung möglichst gut zu widerstehen. Für optisch anspruchsvolle Anwendungen, beispielsweise Deckbeläge von Fussböden, soll eine Beschichtung ausserdem einen hohen Glanzgrad und eine möglichst geringe Neigung zum Vergilben unter Lichteinfluss aufweisen. Härter für Epoxidharze, die auf den im Stand der Technik üblichen (cyclo)aliphatischen Polyaminen wie Isophorondiamin (IPDA), meta-Xylylendiamin (MXDA) oder Trimethylhexamethylendiamin (TMD) in freier Form basieren, haben einen starken Amingeruch und führen aufgrund ihrer Anfälligkeit für Blushing-Effekte oft zu starken Aushärtungsstörungen, insbesondere bei grossflächiger Anwendung wie in Beschichtungen. Als "Blushing-Effekte" werden bei der Aushärtung auftretende Oberflächenmängel wie Trübungen, Flecken, Rauheit und Klebrigkeit bezeichnet, welche durch Salzbildung ("Blushing") von Aminen mit Kohlendioxid (CO₂) aus der Luft verursacht werden und besonders bei hoher Luftfeuchtigkeit und tiefen Temperaturen auftreten. Um das Auftreten von Blushing-Effekten zu verringern, werden die eingesetzten Polyamine typischerweise partiell mit Epoxiden bzw. Epoxidharzen voradduktiert. Dies erhöht aber ihre Viskosität stark und macht den Einsatz von Verdünnern notwendig. Die Verdünner vermindern Blushing-Effekte und verbessern Oberflächenqualität und Sprödigkeit der Beschichtung, werden aber bei der Aushärtung nicht in die Harzmatrix eingebaut und können durch Verdampfungs- oder Diffusionsprozesse freigesetzt werden. Heutzutage werden aber zunehmend emissionsarme Produkte gewünscht, die nach der Aushärtung einen geringen Gehalt an freisetzbaren Substanzen aufweisen. Für emissionsarme Epoxidharz-Zusammensetzungen können Verdünner, wie beispielsweise Benzylalkohol, deshalb nur in geringer Menge oder gar nicht verwendet werden.

Polyamine mit sekundären aliphatischen Aminogruppen als Härter für emissionsarme Epoxidharz-Zusammensetzungen werden bspw. in der EP 2 465 843 A1 offenbart.

### Darstellung der Erfindung

Aufgabe der vorliegenden Erfindung ist es daher, ein niedrigviskoses und geruchsarmes Amin zur Verfügung zu stellen, welches nicht zu Blushing-Effekten neigt und gut verarbeitbare, emissionsarme Epoxidharz-Zusammensetzungen ermöglicht, die schnell und störungsfrei aushärten.

Diese Aufgabe wird durch ein Amin der Formel (I) nach Anspruch 1 gelöst. Das Amin der Formel (I) ist geruchsarm und trotz der Ringstruktur überraschend niedrigviskos. Es weist eine so geringe Reaktivität gegenüber CO₂ auf, dass es an der Luft weder zur Bildung von Krusten noch zu Ausfällungen oder Viskositätserhöhungen neigt. Es weist eine hohe Reaktivität gegenüber Epoxiden auf und ist ausgezeichnet verträglich mit anderen Aminen und mit Epoxidharzen. Mit dem Amin der Formel (I) sind emissionsarme Epoxidharz-Zusammensetzungen zugänglich, welche gut verarbeitbar sind und auch unter ungünstigen Aushärtungsbedingungen, beispielsweise bei 8 °C, rasch aushärten und qualitativ hochstehende Kunststoffe von hoher Härte und ebenmässiger, nichtklebriger Oberfläche mit hohem Glanz bilden.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein Amin der Formel (I), 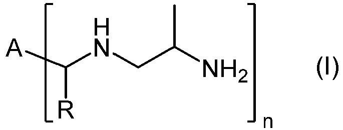 wobei
n für 2 oder 3 steht,
R für einen Wasserstoff-Rest oder für Methyl oder Phenyl steht, und
A für einen, gegebenenfalls Sauerstoff- oder Schwefel- oder Stickstoffatome aufweisenden, n-wertigen Kohlenwasserstoff-Rest mit 5 bis 20 C-Atomen, welcher mindestens einen cycloaliphatischen oder aromatischen Ring enthält, steht.

Mit "Poly" beginnende Substanznamen wie Polyamin, Polyol oder Polyepoxid bezeichnen Substanzen, die formal zwei oder mehr der in ihrem Namen vorkommenden funktionellen Gruppen pro Molekül enthalten.
Als "primäre Aminogruppe" wird eine NH₂-Gruppe bezeichnet, die an einen organischen Rest gebunden ist und als "sekundäre Aminogruppe" wird eine NH-Gruppe bezeichnet, die an zwei organische Reste, welche auch gemeinsam Teil eines Rings sein können, gebunden ist.
Als "Aminwasserstoff" werden die Wasserstoffatome von primären und sekundären Aminogruppen bezeichnet.
Als "Aminwasserstoff-Equivalentgewicht" wird der Gewichtsanteil eines Härters oder eines Amins pro im Härter oder im Amin vorhandenem Aminwasserstoff bezeichnet.
Als "nicht einbaubarer Verdünner" wird eine in einem Epoxidharz lösliche und dessen Viskosität senkende Substanz bezeichnet, welche bei der Aushärtung des Epoxidharzes nicht kovalent in die Harzmatrix eingebaut wird.
Mit dem Begriff "Viskosität" wird im vorliegenden Dokument die dynamische Viskosität oder Scherviskosität bezeichnet, welche durch das Verhältnis zwischen der Schubspannung und der Scherrate (Geschwindigkeitsgefälle) definiert ist und wie in den Ausführungsbeispielen beschrieben bestimmt wird.

Eine gestrichelte Linie in den Formeln in diesem Dokument stellt jeweils die Bindung zwischen einem Substituenten und dem zugehörigen Molekülrest dar. Unter "Molekulargewicht" versteht man im vorliegenden Dokument die molare Masse (in Gramm pro Mol) eines Moleküls. Als "mittleres Molekulargewicht" wird das Zahlenmittel Mₙ einer oligomeren oder polymeren Mischung von Molekülen bezeichnet, welches üblicherweise mittels Gelpermeationschromatographie (GPC) gegen Polystyrol als Standard bestimmt wird.
Als "Raumtemperatur" wird eine Temperatur von 23 °C bezeichnet.

Bevorzugt steht n für 2. Diese Amine sind besonders niedrigviskos.

Bevorzugt steht R für einen Wasserstoff-Rest. Diese Amine sind besonders niedrigviskos und besonders gut herstellbar.

Bevorzugt ist A frei von Hydroxylgruppen. Ein solches Amin der Formel (I) ist besonders niedrigviskos.

Bevorzugt steht A für einen gegebenenfalls substituierten Phenylen- oder Cyclohexylen- oder Dicycloheptylen- oder Tricyclodecylen- oder Pentacyclopentadecylen- oder Furandiyl- oder Tetrahydrofurandiyl- oder Thiophendiyl- oder Tetrahydrothiophendiyl- oder N,N'-Piperazin-bis(2,2-dimethylpropan)diyl-Rest. Diese Amine sind besonders gut zugänglich.

Besonders bevorzugt steht A für einen Phenylen-Rest der Formel (IIa), 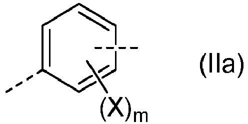 wobei
m für 0 oder 1 oder 2 steht, und
X für gleiche oder verschiedene Reste ausgewählt aus der Gruppe bestehend aus Alkyl und Alkoxy mit jeweils1 bis 4 Kohlenstoffatomen steht.

Weiterhin besonders bevorzugt steht A für einen Cyclohexylen-Rest der Formel (IIb), wobei m und X die bereits genannten Bedeutungen aufweisen.

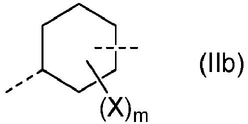

Bevorzugt steht X für Methyl oder Methoxy.
Bevorzugt steht m für 0 oder für 1. Besonders bevorzugt steht m für 0. Diese Amine der Formel (I) sind besonders niedrigviskos.

Weiterhin besonders bevorzugt steht A für einen Furan- oder für einen Thiophen-Rest der Formel (IIc), wobei Z für ein Sauerstoffatom oder ein Schwefelatom steht.

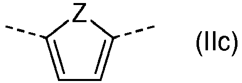

Weiterhin besonders bevorzugt steht A für einen Tetrahydrofuran- oder für einen Tetrahydrothiophen-Rest der Formel (IId), wobei Z die bereits genannten Bedeutungen aufweist.

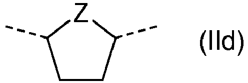

Ganz besonders bevorzugt steht A für einen 1,2- oder 1,3- oder 1,4-Phenylen-Rest oder für einen 1,2- oder 1,3- oder 1,4-Cyclohexylen-Rest.
Insbesondere steht A für einen 1,4-Phenylen-Rest oder für einen 1,4-Cyclohexylen-Rest, am meisten bevorzugt für einen 1,4-Phenylenrest.

Bevorzugt ist das Amin der Formel (I) ausgewählt aus der Gruppe bestehend aus 1,2-Bis(2-aminopropylaminomethyl)benzol, 1,3-Bis(2-aminopropylamino-methyl)benzol, 1,4-Bis(2-aminopropylaminomethyl)benzol, 1,2-Bis(2-amino-propylaminomethyl)cyclohexan, 1,3-Bis(2-aminopropylaminomethyl)cyclohexan, 1,4-Bis(2-aminopropylaminomethyl)cyclohexan, 2(3),5(6)-Bis(2-aminopropylaminomethyl)bicyclo[2.2.1]heptan, 3(4),8(9)-Bis(2-aminopropylaminome-thyl)tricyclo[5.2.1.02,6]decan, 4(5), 11(12)-Bis(2-aminopropylaminomethyl)-pentacyclo[6.5.1.1^{3,6}.0^{2,7}.0^{9,13}]pentadecan, 6,12-Bis(2-aminopropylaminome-thyl)pentacyclo[9.2.1.1^{5,8}.0^{4,9}.0^{2,10}]pentadecan, 2,5-Bis(2-aminopropylamino-methyl)furan, 2,5-Bis(2-aminopropylaminomethyl)tetrahydrofuran, 2,5-Bis(2-aminopropylaminomethyl)thiophen, 2,5-Bis(2-aminopropylaminomethyl)tetrahydrothiophen, N,N'-Bis((N-2-Aminopropyl)-3-amino-2,2-dimethylpropyl)piperazin, 1,2-Bis((N-2-aminopropyl)-1-aminoethyl)benzol, 1,3-Bis((N-2-aminopropyl)-1-aminoethyl)benzol, 1,4-Bis((N-2-aminopropyl)-1-aminoethyl)benzol, 1,2-Bis-((N-2-aminopropyl)-1-aminoethyl)cyclohexan, 1,3-Bis((N-2-aminopropyl)-1-aminoethyl)cyclohexan und 1,4-Bis((N-2-aminopropyl)-1-aminoethyl)cyclohexan.

Davon bevorzugt sind 1,2-Bis(2-aminopropylaminomethyl)benzol, 1,3-Bis(2-aminopropylaminomethyl)benzol, 1,4-Bis(2-aminopropylaminomethyl)benzol, 1,2-Bis(2-aminopropylaminomethyl)cyclohexan, 1,3-Bis(2-aminopropylamino-methyl)cyclohexan, 1,4-Bis(2-aminopropylaminomethyl)cyclohexan, 1,4-Bis((N-2-aminopropyl)-1-aminoethyl)benzol oder 1,4-Bis((N-2-aminopropyl)-1-aminoethyl)cyclohexan.
Besonders bevorzugt ist 1,4-Bis(2-aminopropylaminomethyl)benzol oder 1,4-Bis(2-aminopropylaminomethyl)cyclohexan, insbesondere 1,4-Bis(2-aminopropylaminomethyl)benzol.

Die bevorzugten Amine der Formel (I) zeichnen sich durch besonders niedrige Viskosität und gute Eigenschaften bei der Verwendung als Härter für Epoxidharze aus.

Ein Amin der Formel (I) kann besonders vorteilhaft durch reduktive Alkylierung von 1,2-Propylendiamin mit einer di- oder trifunktionellen Carbonylverbindung der Formel (III) und Wasserstoff erhalten werden. Das Reaktionsprodukt aus dieser Herstellung ist besonders geeignet als Härter für Epoxidharze in der beschriebenen Weise.

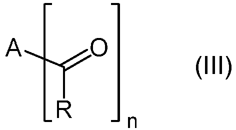

In der Formel (III) weisen n, R und A die bereits genannten Bedeutungen auf.

Ein weiterer Gegenstand der Erfindung ist somit ein Verfahren zur Herstellung eines Amins der Formel (I) durch reduktive Alkylierung von 1,2-Propylendiamin mit mindestens einer di- oder trifunktionellen Carbonylverbindung der Formel (III) und Wasserstoff.
Die reduktive Alkylierung kann direkt mit molekularem Wasserstoff oder indirekt durch Wasserstoff-Transfer von anderen Reagentien, wie beispielsweise Ameisensäure, erfolgen. Bevorzugt wird molekularer Wasserstoff verwendet. Dabei werden die Bedingungen vorteilhaft so gewählt, dass vor allem jeweils eine primäre Aminogruppe von 1,2-Propylendiamin mit guter Selektivität einfach alkyliert wird und sich somit zwei oder drei 1,2-Propylendiamin-Moleküle und die Carbonylverbindung der Formel (III) zu einem Amin der Formel (I) vereinen. Für den Fall, dass die Carbonylverbindung der Formel (III) einen Rest A mit einem aromatischen Ring enthält, kann dieser, je nach Einstellung der Reaktionsbedingungen bei der reduktiven Alkylierung, unhydriert bleiben oder gezielt mithydriert werden.
Bevorzugt wird die Umsetzung bei einer Temperatur von 40 bis 120 °C und in Anwesenheit eines geeigneten Katalysators durchgeführt. Als Katalysator bevorzugt sind Palladium auf Kohle (Pd/C), Platin auf Kohle (Pt/C), Adams-Katalysator oder Raney-Nickel, insbesondere Palladium auf Kohle oder Raney-Nickel.
Bei Verwendung von molekularem Wasserstoff wird bevorzugt in einer Druckapparatur bei einem Wasserstoff-Druck von 5 bis 250 bar, insbesondere 10 bis 100 bar, gearbeitet.

In einer bevorzugten Ausführungsform der Herstellung wird 1,2-Propylendiamin gegenüber den Carbonylgruppen der Carbonylverbindung der Formel (III) im stöchiometrischen Überschuss eingesetzt. Das Verhältnis zwischen der Anzahl 1,2-Propylendiamin-Moleküle und der Anzahl Carbonylgruppen beträgt bevorzugt mindestens 2/1, insbesondere mindestens 3/1, besonders bevorzugt mindestens 4/1. Das überschüssige 1,2-Propylendiamin wird vor oder bevorzugt nach der Reduktion entfernt, insbesondere durch Destillation.
Die Herstellung des Amins der Formel (I) durch reduktive Alkylierung auf die beschriebene Weise ist für die Verwendung als Härter für Epoxidharze besonders vorteilhaft, da primäre Aminogruppen mit guter Selektivität einfach alkyliert werden, während sekundäre Aminogruppen kaum weiter alkyliert werden. So hergestellte Amine der Formel (I) sind besonders rein, weisen eine besonders niedrige Viskosität auf und können ohne weitere Aufarbeitung als Härter für Epoxidharze verwendet werden. Das Produkt aus dem beschriebenen Herstellverfahren kann deshalb ohne weitere Aufbereitung zur Aushärtung von Epoxidharzen in der beschriebenen Weise verwendet werden.
Je nach Reaktionsbedingungen bei der Herstellung kann das Produkt nach der Herstellung zusätzlich zum Amin der Formel (I) Nebenprodukte enthalten. Insbesondere können oligomere Verbindungen aus der Dialkylierung von 1,2-Propylendiamin enthalten sein. Solche oligomeren Verbindungen führen zu einer Erhöhung der Viskosität des Reaktionsproduktes. Die Herstellung wird bevorzugt so geführt, dass die Bildung von oligomeren Verbindungen möglichst unterdrückt wird. Weiterhin können als Nebenprodukte an der weniger reaktiven Aminogruppe alkylierte 1,2-Propylendiamine, beispielsweise der Formel 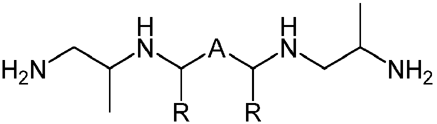 oder der Formel 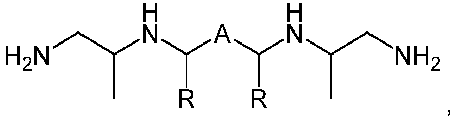 enthalten sein.

Als Carbonylverbindung der Formel (III) geeignet sind insbesondere
- Dialdehyde, insbesondere ortho-Phthalaldehyd (1,2-Benzoldicarbaldehyd), Isophthalaldehyd (1,3-Benzoldicarbaldehyd), Terephthalaldehyd (1,4-Benzoldicarbaldehyd), Naphthalindicarboxaldehyd, Anthracendicarboxaldehyd, 2,5-Furandicarbaldehyd, 2,5-Thiophendicarbaldehyd, Cyclopentandicarbaldehyd, 1,2-Cyclohexandicarbaldehyd, 1,3-Cyclohexandicarbaldehyd, 1,4-Cyclohexandicarbaldehyd, 2(3),5(6)-Diformyl-bicyclo[2.2.1]heptan (Norbornandicarbaldehyd), 3(4),8(9)-Diformyl-tricyclo[5.2.1.02,6]decan (Tricyclodecandicarbaldehyd oder TCD-Dialdehyd), 4(5),11(12)-Diformyl-pentacyclo-[6.5.1.1^{3,6}.0^{2,7}.0^{9,13}]pentadecan bzw. 6,12-Diformyl-pentacyclo-[9.2.1.1^{5,8}.0^{4,9}.0^{2,10}]pentadecan (Pentacyclopentadecandicarbaldehyd), 2,5-Tetrahydrofurandicarbaldehyd, 2,5-Tetrahydrothiophendicarbaldehyd, 1,3-Bis(4,4-dimethyl-5-oxo-2-pentyl)benzol, 1,4-Bis(4,4-dimethyl-5-oxo-2-pentyl)benzol, 3-(3-Oxopropyl)cyclohexancarbaldehyd, 4-(3-Oxopropyl)cyclohexancarbaldehyd, 3-(1-Formylethyl)cyclohexancarbaldehyd, 4-(1-Formylethyl)cyclohexancarbaldehyd, N,N'-Bis(2,2-dimethyl-3-oxopropyl)piperazin, N,N'-Bis(2,2-diethyl-3-oxopropyl)piperazin, N,N'-Bis(2-methyl-2-propyl-3-oxopropyl)piperazin oder N,N'-Bis(2-butyl-2-ethyl-3-oxopropyl)piperazin;
- Trialdehyde, insbesondere 1,3,5-Benzoltricarbaldehyd oder 1,3,5-Cyclohexantricarbaldehyd;
- Diketone, insbesondere 1,2-Diacetylbenzol, 1,3-Diacetylbenzol, 1,4-Diacetylbenzol, 1,2-Diacetylcyclohexan, 1,3-Diacetylcyclohexan oder 1,4-Diacetylcyclohexan;
- Triketone, insbesondere 1,3,5-Triacetylbenzol oder 1,3,5-Triacetylcyclohexan; oder
- Ketoaldehyde, insbesondere 2-Acetylbenzaldehyd, 3-Acetylbenzaldehyd oder 4-Acetylbenzaldehyd.
Bevorzugt sind die Dialdehyde, insbesondere ortho-Phthalaldehyd, Isophthalaldehyd, Terephthalaldehyd, 2,5-Furandicarbaldehyd, 2,5-Thiophendicarbaldehyd, 1,2-Cyclohexandicarbaldehyd, 1,3-Cyclohexandicarbaldehyd, 1,4-Cyclohexandicarbaldehyd, Norbornandicarbaldehyd, TCD-Dialdehyd, Pentacyclopentadecandicarbaldehyd oder N,N'-Bis(2,2-dimethyl-3-oxopropyl)-piperazin, sowie die Diketone, insbesondere 1,2-Diacetylbenzol, 1,3-Diacetylbenzol, 1,4-Diacetylbenzol, 1,2-Diacetylcyclohexan, 1,3-Diacetylcyclohexan oder 1,4-Diacetylcyclohexan.
Besonders bevorzugt sind ortho-Phthalaldehyd, Isophthalaldehyd, Terephthalaldehyd, 1,2-Cyclohexandicarbaldehyd, 1,3-Cyclohexandicarbaldehyd, 1,4-Cyclohexandicarbaldehyd, 1,4-Diacetylbenzol oder 1,4-Diacetylcyclohexan.

Am meisten bevorzugt sind Terephthalaldehyd oder 1,4-Cyclohexandicarbaldehyd, insbesondere Terephthalaldehyd.

Es kann vorteilhaft sein, das Reaktionsprodukt aus den beschriebenen Herstellungen mittels Destillation zu reinigen. Auf diese Weise können nicht umgesetztes 1,2-Propylendiamin und/oder oligomere Nebenprodukte aus der Reaktionsmischung entfernt werden.

Das Amin der Formel (I) ist eine geruchsarme Substanz, die trotz der Ringstruktur eine überraschend niedrige Viskosität aufweist. Es weist eine so geringe Reaktivität gegenüber CO₂ auf, dass es - im Gegensatz zu vielen aus dem Stand der Technik bekannten Aminen - an der Luft weder zur Bildung von Krusten noch zu Ausfällungen oder Viskositätserhöhungen neigt. Es weist eine hohe Reaktivität gegenüber Epoxiden auf und zeigt eine ausgezeichnete Verträglichkeit mit anderen Aminen und mit Epoxidharzen und ist somit besonders geeignet für die Verwendung als Härter für Epoxidharze.

Ein weiterer Gegenstand der Erfindung ist dementsprechend die Verwendung eines Amins der Formel (I) als Härter für Epoxidharze.
Insbesondere wird ein Reaktionsprodukt aus der reduktiven Alkylierung von 1,2-Propylendiamin mit einer di- oder trifunktionellen Carbonylverbindung der Formel (III) und Wasserstoff, wie vorgängig beschrieben, als Härter für Epoxidharze verwendet. Ein solches Reaktionsprodukt ist einfach zugänglich und enthält einen hohen Anteil an Amin der Formel (I).
Typischerweise enthält ein solches Reaktionsprodukt neben dem Amin der Formel (I) auch am N²-Stickstoff von 1,2-Propylendiamin alkylierte Anteile, wie beispielsweise 1-(2-Aminopropylaminomethyl)-4-(1-aminoprop-2-ylaminomethyl)benzol, 1,4-Bis(1-aminoprop-2-ylaminomethyl)benzol, 1-(2-Aminopropyl-aminomethyl)-4-(1-aminoprop-2-ylaminomethyl)cyclohexan, 1,4-Bis(1-amino-prop-2-ylaminomethyl)cyclohexan, oder oligomere Reaktionsprodukte.

Ein weiterer Gegenstand der Erfindung ist ein Härter für Epoxidharze enthaltend mindestens ein Amin der Formel (I) und mindestens ein weiteres Amin und/oder mindestens einen Beschleuniger. Das weitere Amin ist dabei kein Amin der Formel (I). Ein solcher Härter weist eine besonders niedrige Viskosität und/oder eine besonders hohe Reaktivität gegenüber dem Epoxidharz auf.

Als Beschleuniger geeignet sind Substanzen, welche die Reaktion zwischen Aminogruppen und Epoxidgruppen beschleunigen, insbesondere Säuren oder zu Säuren hydrolysierbare Verbindungen, insbesondere organische Carbonsäuren wie Essigsäure, Benzoesäure, Salicylsäure, 2-Nitrobenzoesäure, Milchsäure, organische Sulfonsäuren wie Methansulfonsäure, p-Toluolsulfonsäure oder 4-Dodecylbenzolsulfonsäure, Sulfonsäureester, andere organische oder anorganische Säuren wie insbesondere Phosphorsäure, oder Mischungen der vorgenannten Säuren und Säureester; tertiäre Amine wie insbesondere 1,4-Diazabicyclo[2.2.2]octan, Benzyldimethylamin, α-Methylbenzyldimethylamin, Triethanolamin, Dimethyl-aminopropylamin, Imidazole wie insbesondere N-Methylimidazol, N-Vinylimidazol oder 1,2-Dimethylimidazol, Salze solcher tertiärer Amine, quaternäre Ammoniumsalze, wie insbesondere Benzyltrimethylammoniumchlorid, Amidine wie insbesondere 1,8-Diazabicyclo[5.4.0]undec-7-en, Guanidine wie insbesondere 1,1,3,3-Tetramethylguanidin, Phenole, insbesondere Bisphenole, Phenol-Harze oder Mannich-Basen wie insbesondere 2-(Dimethylaminomethyl)phenol, 2,4,6-Tris(dimethylaminomethyl)phenol oder Polymere aus Phenol, Formaldehyd und N,N-Dimethyl-1,3-propandiamin, Phosphite wie insbesondere Di- oder Triphenylphosphite, oder Mercaptogruppen aufweisende Verbindungen. Als Beschleuniger bevorzugt sind Säuren, tertiäre Amine oder Mannich-Basen.
Am meisten bevorzugt ist Salicylsäure oder 2,4,6-Tris(dimethylaminomethyl)-phenol oder eine Kombination davon.

Als weiteres Amin geeignet sind insbesondere Polyamine, welche mindestens zwei, insbesondere mindestens drei, gegenüber Epoxidgruppen reaktive Aminwasserstoffe aufweisen, insbesondere die folgenden Polyamine:
- Reaktionsprodukte aus der reduktiven Alkylierung von 1,2-Propylendiamin mit einer di- oder trifunktionellen Carbonylverbindung der Formel (III) und Wasserstoff, welche nicht der Formel (I) entsprechen, insbesondere 1-(2-Aminopropylaminomethyl)-4-(1-aminoprop-2-ylaminomethyl)benzol, 1,4-Bis(1-aminoprop-2-ylaminomethyl)benzol, 1-(2-Aminopropylaminomethyl)-4-(1-aminoprop-2-ylaminomethyl)cyclohexan, 1,4-Bis(1-aminoprop-2-ylaminomethyl)cyclohexan oder oligomere Reaktionsprodukte;
- aliphatische, cycloaliphatische oder arylaliphatische primäre Diamine, insbesondere 2,2-Dimethyl-1,3-propandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2(4),4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- oder 1,4-Diaminocyclohexan, Bis(4-aminocyclohexyl)methan (H₁₂-MDA), Bis(4-amino-3-methylcyclohexyl)methan, Bis(4-amino-3-ethylcyclohexyl)methan, Bis(4-amino-3,5-dimethylcyclohexyl)methan, Bis(4-amino-3-ethyl-5-methylcyclohexyl)methan, 1-Amino-3-aminomethyl-3,5,5-trimethylcyclohexan (Isophorondiamin oder IPDA), 2- oder 4-Methyl-1,3-diaminocyclohexan oder Mischungen davon, 1,3-Bis(aminomethyl)cyclohexan, 1,4-Bis(aminomethyl)cyclohexan, 2,5(2,6)-Bis(aminomethyl)bicyclo[2.2.1]heptan (NBDA), 3(4),8(9)-Bis(aminomethyl)tricyclo-[5.2.1.0^{2,6}]decan, 1,4-Diamino-2,2,6-trimethylcyclohexan (TMCDA), 1,8-Menthandiamin, 3,9-Bis(3-aminopropyl)-2,4,8,10-tetraoxaspiro[5.5]undecan, 1,3-Bis(aminomethyl)benzol (MXDA) oder 1,4-Bis(aminomethyl)benzol;
- aliphatische, cycloaliphatische oder arylaliphatische primäre Triamine, insbesondere 4-Aminomethyl-1,8-octandiamin, 1,3,5-Tris(aminomethyl)benzol, 1,3,5-Tris(aminomethyl)cyclohexan, Tris(2-aminoethyl)amin, Tris(2-aminopropyl)amin oder Tris(3-aminopropyl)amin;
- Ethergruppen-haltige aliphatische primäre Di- oder Triamine, insbesondere Bis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin oder höhere Oligomere dieser Diamine, Bis(3-aminopropyl)polytetrahydrofurane oder andere Polytetrahydrofurandiamine, cycloaliphatische Ethergruppen-haltige Diamine aus der Propoxylierung und nachfolgenden Aminierung von 1,4-Dimethylolcyclohexan, erhältlich insbesondere als Jeffamine® RFD-270 (von Huntsman), oder Polyoxyalkylendi- oder -triamine, welche typischerweise Produkte aus der Aminierung von Polyoxyalkylendi- oder -triolen darstellen und beispielsweise erhältlich sind unter dem Namen Jeffamine® (von Huntsman), unter dem Namen Polyetheramine (von BASF) oder unter dem Namen PC Amine® (von Nitroil). Insbesondere geeignete Polyoxyalkylendi- oder -triamine sind Jeffamine® D-230, Jeffamine® D-400, Jeffamine® D-2000, Jeffamine® EDR-104, Jeffamine® EDR-148, Jeffamine® EDR-176, Jeffamine® T-403, Jeffamine® T-3000, Jeffamine® T-5000, oder entsprechende Amine von BASF oder Nitroil;
- sekundäre Aminogruppen aufweisende Polyamine mit zwei primären aliphatischen Aminogruppen, wie insbesondere 3-(2-Aminoethyl)aminopropylamin, Bis(hexamethylen)triamin (BHMT), Diethylentriamin (DETA), Triethylentetramin (TETA), Tetraethylenpentamin (TEPA), Pentaethylenhexamin (PEHA) oder höhere Homologe linearer Polyethylenamine wie Polyethylenpolyamin mit 5 bis 7 Ethylenamin-Einheiten (sogenanntes "higher ethylenepolyamine", HEPA), Produkte aus der mehrfachen Cyanoethylierung oder Cyanobutylierung und anschliessender Hydrierung von primären Di- und Polyaminen mit mindestens zwei primären Aminogruppen, wie Dipropylentriamin (DPTA), N-(2-Aminoethyl)-1,3-propandiamin (N3-Amin), N,N'-Bis(3-aminopropyl)ethylendiamin (N4-Amin), N,N'-Bis(3-aminopropyl)-1,4-diaminobutan, N5-(3-Aminopropyl)-2-methyl-1,5-pentandiamin, N3-(3-Aminopentyl)-1,3-pentandiamin, N5-(3-Amino-1-ethylpropyl)-2-methyl-1,5-pentandiamin oder N,N'-Bis(3-amino-1-ethylpropyl)-2-methyl-1,5-pentandiamin;
- Polyamine mit ein oder zwei sekundären Aminogruppen, insbesondere Produkte aus der reduktiven Alkylierung von primären aliphatischen Polyaminen mit monofunktionellen Aldehyden oder Ketonen, insbesondere N-Benzyl-1,3-bis(aminomethyl)benzol, N,N'-Dibenzyl-1,3-bis(aminomethyl)benzol, N-2-Ethylhexyl-1,3-bis(aminomethyl)benzol, N,N'-Bis(2-ethylhexyl)-1,3-bis(aminomethyl)benzol, N-Benzyl-1,2-propandiamin, N-(4-Methoxybenzyl)-1,2-propandiamin, N-(4-(Dimethylamino)benzyl)-1,2-propandiamin, N-(1-Phenylethyl)-1,2-propandiamin, N-Benzhydryl-1,2-propandiamin, N-(1-(4'-Methyl)phenylethyl)-1,2-propandiami, N-(1-(4'-Methoxy)phenylethyl)-1,2-propandiamin oder partiell styrolisierte Polyamine wie zum Beispiel styrolisiertes MXDA (erhältlich als Gaskamine® 240 von Mitsubishi Gas Chemical);
- aromatische Polyamine, wie insbesondere m- und p-Phenylendiamin, 4,4'-, 2,4' und/oder 2,2'-Diaminodiphenylmethan, 3,3'-Dichloro-4,4'-diaminodiphenylmethan (MOCA), 2,4- und/oder 2,6-Toluylendiamin, Mischungen von 3,5-Dimethylthio-2,4- und -2,6-toluylendiamin (erhältlich als Ethacure® 300 von Albermarle), Mischungen von 3,5-Diethyl-2,4- und -2,6-toluylendiamin (DETDA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 4,4'-Diaminodiphenylsulfon (DDS), 4-Amino-N-(4-aminophenyl)benzolsulfonamid, 5,5'-Methylendianthranilsäure, Dimethyl-(5,5'-methylendianthranilat), 1,3-Propylen-bis(4-aminobenzoat), 1,4-Butylen-bis(4-aminobenzoat), Polytetramethylenoxid-bis(4-aminobenzoat) (erhältlich als Versalink® von Air Products), 1,2-Bis(2-aminophenylthio)ethan, 2-Methylpropyl-(4-chloro-3,5-diaminobenzoat) oder tert.Butyl-(4-chloro-3,5-diaminobenzoat);
- Addukte der genannten Polyamine mit Epoxiden oder Epoxidharzen, insbesondere Addukte mit Diepoxiden im Molverhältnis von ungefähr 2/1, Addukte mit Monoepoxiden im Molverhältnis von ungefähr 1/1, oder Umsetzungsprodukte aus Aminen und Epichlorhydrin, insbesondere jenes von 1,3-Bis(aminomethyl)benzol, kommerziell erhältlich als Gaskamine® 328 (von Mitsubishi Gas Chemical);
- Polyamidoamine, insbesondere Umsetzungsprodukte aus einer ein- oder mehrwertigen Carbonsäure, beziehungsweise deren Ester oder Anhydride, insbesondere einer Dimerfettsäure, mit einem im stöchiometrischen Überschuss eingesetzten aliphatischen, cycloaliphatischen oder aromatischen Polyamin, insbesondere einem Polyalkylenamin wie beispielsweise DETA oder TETA, insbesondere die kommerziell erhältlichen Polyamidoamine Versamid® 100, 125, 140 oder 150 (von Cognis), Aradur® 223, 250 oder 848 (von Huntsman), Euretek® 3607 oder 530 (von Huntsman) oder Beckopox® EH 651, EH 654, EH 655, EH 661 oder EH 663 (von Cytec); oder
- Phenalkamine, auch Mannich-Basen genannt, insbesondere Umsetzungsprodukte aus einer Mannich-Reaktion von Phenolen, insbesondere Cardanol, mit Aldehyden, insbesondere Formaldehyd, insbesondere die kommerziell erhältlichen Phenalkamine Cardolite® NC-541, NC-557, NC-558, NC-566, Lite 2001 oder Lite 2002 (von Cardolite), Aradur® 3440, 3441, 3442 oder 3460 (von Huntsman) oder Beckopox® EH 614, EH 621, EH 624, EH 628 oder EH 629 (von Cytec).

Als weiteres Amin bevorzugt sind Reaktionsprodukte aus der reduktiven Alkylierung von 1,2-Propylendiamin mit einer di- oder trifunktionellen Carbonylverbindung der Formel (III) und Wasserstoff, welche nicht der Formel (I) entsprechen, insbesondere 1-(2-Aminopropylaminomethyl)-4-(1-aminoprop-2-ylaminomethyl)benzol, 1,4-Bis(1-aminoprop-2-ylaminomethyl)benzol, 1-(2-Aminopropylaminomethyl)-4-(1-aminoprop-2-ylaminomethyl)cyclohexan, 1,4-Bis(1-aminoprop-2-ylaminomethyl)cyclohexan, oder oligomere Reaktionsprodukte.

Als weiteres Amin bevorzugt sind weiterhin partiell styrolisierte Polyamine, insbesondere partiell styrolisiertes MXDA, oder Produkte aus der reduktiven Alkylierung von primären aliphatischen Polyaminen mit monofunktionellen Aldehyden oder Ketonen, insbesondere solche von 1,2-Propylendiamin wie insbesondere N-Benzyl-1,2-propandiamin, N-(4-Methoxybenzyl)-1,2-propandiamin, N-(4-(Dimethylamino)benzyl)-1,2-propandiamin, N-(1-Phenylethyl)-1,2-propandiamin, N-Benzhydryl-1,2-propandiamin, N-(1-(4'-Methyl)phenylethyl)-1,2-propandiami oder N-(1-(4'-Methoxy)phenylethyl)-1,2-propandiamin.

Als weiteres Amin bevorzugt sind weiterhin Ethergruppen-haltige aliphatische primäre Di- oder Triamine, insbesondere Polyoxyalkylendi- oder -triamine mit einem mittleren Molekulargewicht im Bereich von 200 bis 500 g/mol, insbesondere Jeffamine® D-230 oder Jeffamine® T-403 (beide von Huntsman), oder cycloaliphatische Ethergruppen-haltige Diamine aus der Propoxylierung und nachfolgenden Aminierung von 1,4-Dimethylolcyclohexan, insbesondere Jeffamine® RFD-270 (von Huntsman). Ein solches Amin ermöglicht Epoxidharz-Zusammensetzungen mit einer zuverlässigen Aushärtung zu hoher Endhärte ohne sogenanntes "Einfrieren" und mit geringer Sprödigkeit nach der Aushärtung. Mit "Einfrieren" wird das Phänomen bezeichnet, dass eine Epoxidharz-Zusammensetzung bei einer gegebenen Temperatur nach anfänglich guter Härteentwicklung nicht zu der erwarteten Endhärte aushärtet, sondern die Aushärtung bei einer geringeren Härte stehenbleibt. Solche Effekte treten insbesondere bei tiefen Aushärtungstemperaturen auf.

Als weiteres Amin bevorzugt sind weiterhin Addukte aus (i) mindestens einem Polyamin mit mindestens drei gegenüber Epoxidgruppen reaktiven Aminwasserstoffen mit (ii) mindestens einem Epoxid.
Als Polyamin für ein solches Addukt bevorzugt sind die vorgängig genannten Polyamine mit mindestens drei gegenüber Epoxidgruppen reaktiven Aminwasserstoffen, oder kleinere Polyamine wie insbesondere Ethylendiamin, die isomeren Propylendiamine oder die isomeren Butylendiamine.
Als Epoxid für ein solches Addukt bevorzugt sind Diepoxide oder Monoepoxide, insbesondere aromatische Monoepoxide wie insbesondere Kresylglycidylether, tert.Butylphenylglycidylether oder der Glycidylether von Cardanol. Besonders bevorzugt ist Kresylglycidylether. Als Kresylglycidylether geeignet sind alle isomeren Kresylglycidylether oder Gemische davon, insbesondere kommerziell erhältiche Typen wie insbesondere Araldite® DY-K (von Huntsman), Polypox™ R6 (von Dow), Heloxy™ KR (von Hexion) oder Erisys® GE-10 (von CVC Spec. Chem.).
Das Addukt wird bevorzugt hergestellt durch langsames Zudosieren des Epoxids zu vorgelegtem Polyamin, wobei die Temperatur der Reaktanden bevorzugt im Bereich von 40 bis 120 °C, insbesondere 50 bis 110 °C, gehalten wird.

Ein bevorzugtes Addukt ist ein Addukt aus 1,2-Propylendiamin mit Kresylglycidylether, welches inbesondere hergestellt ist mit einem Überschuss 1,2-Propylendiamin und nachfolgender Entfernung des Überschusses mittels Destillation.
Ein weiteres bevorzugtes Addukt ist ein Addukt aus 1,5-Diamino-2-methylpentan mit Kresylglycidylether, welches inbesondere entweder hergestellt ist mit einem Überschuss 1,5-Diamino-2-methylpentan und nachfolgender Entfernung des Überschusses mittels Destillation, oder mit einem leichten Überschuss an Kresylglycidylether.
Ein weiteres bevorzugtes Addukt ist ein Addukt aus 2,2(4),4-Trimethylhexamethylendiamin mit Kresylglycidylether, welches inbesondere hergestellt ist mit einem leichten Überschuss an 2,2(4),4-Trimethylhexamethylendiamin.
Der Begriff "Überschuss" bezieht sich dabei nicht auf die Reaktivgruppen, sondern auf das Molverhältnis zwischen dem Polyaminmolekül und dem Monoepoxidmolekül.
Diese bevorzugten Addukte sind vergleichsweise niedrigviskos, weisen eine besonders gute Verträglichkeit und Reaktivität mit den üblichen Epoxidharz-Zusammensetzungen auf und ermöglichen ausgehärtete Filme von hohem Glanz und hoher Härte.

Der erfindungsgemässe Härter enthält bevorzugt 1 bis 95 Gewichts-%, besonders bevorzugt 2 bis 90 Gewichts-%, insbesondere 5 bis 80 Gewichts-%, Amin der Formel (I).
Solche Härter zeichnen sich durch eine niedrige Viskosität aus und ermöglichen Epoxidharz-Beschichtungen mit hoher Aushärtungsgeschwindigkeit, kaum Neigung zu Blushing-Effekten und hoher Härte.

Typischerweise enthält der Härter einen gewissen Anteil an am N²-Stickstoff alkylierten Produkten, beispielsweise im Fall von 1,4-Bis(2-aminopropylaminomethyl)benzol einen gewissen Anteil an 1-(2-Aminopropylaminomethyl)-4-(1-aminoprop-2-ylaminomethyl)benzol und/oder 1,4-Bis(1-aminoprop-2-ylaminomethyl)benzol.

Der Härter ist bevorzugt weitgehend frei von 1,2-Propylendiamin. Er enthält insbesondere weniger als 1 Gewichts-%, besonders bevorzugt weniger als 0.1 Gewichts-%, 1,2-Propylendiamin.
Weiterhin bevorzugt ist der Härter weitgehend frei von Aminen mit einem Molekulargewicht unterhalb von 120 g/mol, insbesondere unterhalb von 150 g/mol. Bevorzugt enthält der Härter weniger als 2 Gewichts-%, insbesondere weniger als 1 Gewichts-%, Amine mit einem Molekulargewicht unterhalb von 120 g/mol, insbesondere unterhalb von 150 g/mol.
Ein solcher Härter ist toxikologisch und geruchlich besonders vorteilhaft und ermöglicht Beschichtungen mit besonders schönen Oberflächen.

Der Härter kann weiterhin mindestens einen nicht einbaubaren Verdünner enthalten, insbesondere Xylol, 2-Methoxyethanol, Dimethoxyethanol, 2-Ethoxyethanol, 2-Propoxyethanol, 2-Isopropoxyethanol, 2-Butoxyethanol, 2-Phenoxyethanol, 2-Benzyloxyethanol, Benzylalkohol, Ethylenglykol, Ethylenglykoldimethylether, Ethylenglykoldiethylether, Ethylenglykoldibutylether, Ethylenglykoldiphenylether, Diethylenglykol, Diethylenglykol-monomethylether, Diethylenglykol-monoethylether, Diethylenglykol-mono-n-butylether, Diethylenglykoldimethylether, Diethylenglykoldiethylether, Diethylenglykoldi-n-butylylether, Propylenglykolbutylether, Propylenglykolphenylether, Dipropylenglykol, Dipropylenglykolmonomethylether, Dipropylenglykoldimethylether, Dipropylenglykoldi-n-butylether, N-Methylpyrrolidon, Diphenylmethan, Diisopropylnaphthalin, Erdölfraktionen wie zum Beispiel Solvesso®-Typen (von Exxon), Alkylphenole wie tert.Butylphenol, Nonylphenol, Dodecylphenol und 8,11,14-Pentadecatrienylphenol (Cardanol, aus Cashewschalen-Öl, erhältlich beispielsweise als Cardolite NC-700 von Cardolite Corp., USA), styrolisiertes Phenol, Bisphenole, aromatische Kohlenwasserstoffharze, insbesondere Phenolgruppen-haltige Typen, alkoxyliertes Phenol, insbesondere ethoxyliertes oder propoxyliertes Phenol, insbesondere 2-Phenoxyethanol, Adipate, Sebacate, Phthalate, Benzoate, organische Phosphor- oder Sulfonsäureester oder Sulfonamide. Bevorzugt sind Benzylalkohol, Dodecylphenol, tert.Butylphenol, styrolisiertes Phenol, ethoxyliertes Phenol oder phenolgruppenhaltige aromatische Kohlenwasserstoffharze, insbesondere die Novares®-Typen LS 500, LX 200, LA 300 oder LA 700 (von Rütgers).

Bevorzugt enthält der Härter keinen oder nur einen geringen Gehalt an nicht einbaubaren Verdünnern. Bevorzugt enthält der Härter maximal 10 Gewichts-%, insbesondere maximal 5 Gewichts-%, nicht einbaubare Verdünner.

Der Härter kann weitere gegenüber Epoxidgruppen reaktive Substanzen enthalten, beispielsweise Monoamine wie Hexylamin oder Benzylamin, oder Mercaptogruppen aufweisende Verbindungen, insbesondere die Folgenden:
- flüssige Mercaptan-terminierte Polysulfid-Polymere, bekannt unter dem Markennamen Thiokol® (von Morton Thiokol; beispielsweise erhältlich von SPI Supplies, oder von Toray Fine Chemicals), insbesondere die Typen LP-3, LP-33, LP-980, LP-23, LP-55, LP-56, LP-12, LP-31, LP-32 oder LP-2; sowie weiterhin bekannt unter dem Markennamen Thioplast® (von Akzo Nobel), insbesondere die Typen G 10, G 112, G 131, G 1, G 12, G 21, G 22, G 44 oder G 4;
- Mercaptan-terminierte Polyoxyalkylen-Ether, erhältlich beispielsweise durch Umsetzung von Polyoxyalkylendi- oder -triolen entweder mit Epichlorhydrin oder mit einem Alkylenoxid, gefolgt von Natriumhydrogensulfid;
- Mercaptan-terminierte Verbindungen in Form von Polyoxyalkylen-Derivaten, bekannt unter dem Markennamen Capcure® (von Cognis), insbesondere die Typen WR-8, LOF oder 3-800;
- Polyester von Thiocarbonsäuren, beispielsweise Pentaerythritoltetramercaptoacetat, Trimethylolpropantrimercaptoacetat, Glykoldimercaptoacetat, Pentaerythritoltetra-(3-mercaptopropionat), Trimethylolpropantri(3-mercaptopropionat) oder Glykoldi-(3-mercaptopropionat), oder Veresterungsprodukte von Polyoxyalkylendiolen oder -triolen, ethoxyliertem Trimethylolpropan oder Polyester-Diolen mit Thiocarbonsäuren wie Thioglykolsäure oder 2- oder 3-Mercaptopropionsäure; oder
- weitere Mercaptogruppen aufweisende Verbindungen, wie insbesondere 2,4,6-Trimercapto-1,3,5-triazin, 2,2'-(Ethylendioxy)-diethanthiol (Triethylenglykol-dimercaptan) oder Ethandithiol.

Ein weiterer Gegenstand der Erfindung ist eine Epoxidharz-Zusammensetzung umfassend
- eine Harz-Komponente enthaltend mindestens ein Epoxidharz und
- eine Härter-Komponente enthaltend mindestens ein Amin der Formel (I) wie vorgängig beschrieben.

Bevorzugt enthält die Härter-Komponente einen Härter enthaltend mindestens ein Amin der Formel (I) und mindestens ein weiteres Amin und/oder mindestens einen Beschleuniger, wie vorgängig beschrieben.

Als Epoxidharz sind übliche technische Epoxidharze geeignet. Diese werden auf bekannte Art und Weise erhalten, zum Beispiel aus der Oxidation der entsprechenden Olefine oder aus der Reaktion von Epichlorhydrin mit den entsprechenden Polyolen, Polyphenolen oder Aminen.
Als Epoxidharz besonders geeignet sind sogenannte Polyepoxid-Flüssigharze, im folgenden als "Flüssigharz" bezeichnet. Diese weisen eine Glasübergangstemperatur unterhalb von 25°C auf.
Ebenfalls möglich als Epoxidharz sind sogenannte Festharze, welche eine Glasübergangstemperatur oberhalb von 25°C aufweisen und sich zu bei 25°C schüttfähigen Pulvern zerkleinern lassen.

Geeignete Epoxidharze sind insbesondere aromatische Epoxidharze, insbesondere die Glycidylisierungsprodukte von:
- Bisphenol-A, Bisphenol-F oder Bisphenol-A/F, wobei A für Aceton und F für Formaldehyd steht, welche als Edukte zur Herstellung dieser Bisphenole dienten. Im Fall von Bisphenol-F können auch Stellungsisomere vorhanden sein, insbesondere abgeleitet von 2,4'- oder 2,2'-Hydroxyphenylmethan.
- Dihydroxybenzol-Derivaten wie Resorcin, Hydrochinon oder Brenzkatechin;
- weiteren Bisphenolen oder Polyphenolen wie Bis(4-hydroxy-3-methylphenyl)methan, 2,2-Bis(4-hydroxy-3-methylphenyl)propan (Bisphenol-C), Bis-(3,5-dimethyl-4-hydroxyphenyl)methan, 2,2-Bis(3,5-dimethyl-4-hydroxyphenyl)propan, 2,2-Bis(3,5-dibromo-4-hydroxyphenyl)propan, 2,2-Bis(4-hydroxy-3-tert.butylphenyl)propan, 2,2-Bis(4-hydroxyphenyl)butan (Bisphenol-B), 3,3-Bis(4-hydroxyphenyl)pentan, 3,4-Bis(4-hydroxyphenyl)hexan, 4,4-Bis(4-hydroxyphenyl)heptan, 2,4-Bis(4-hydroxyphenyl)-2-methylbutan, 2,4-Bis-(3,5-dimethyl-4-hydroxyphenyl)-2-methylbutan, 1,1-Bis(4-hydroxyphenyl)-cyclohexan (Bisphenol-Z), 1,1-Bis(4-hydroxyphenyl)-3,3,5-trimethylcyclohexan (Bisphenol-TMC), 1,1-Bis(4-hydroxyphenyl)-1-phenylethan, 1,4-Bis[2-(4-hydroxyphenyl)-2-propyl]benzol (Bisphenol-P), 1,3-Bis[2-(4-hydroxyphenyl)-2-propyl]benzol (Bisphenol-M), 4,4'-Dihydroxydiphenyl (DOD), 4,4'-Dihydroxybenzophenon, Bis(2-hydroxynaphth-1-yl)methan, Bis(4-hydroxynaphth-1-yl)methan, 1,5-Dihydroxynaphthalin, Tris(4-hydroxyphenyl)methan, 1,1,2,2-Tetrakis(4-hydroxyphenyl)ethan, Bis(4-hydroxyphenyl)ether oder Bis(4-hydroxyphenyl)sulfon;
- Kondensationsprodukten von Phenolen mit Formaldehyd, die unter sauren Bedingungen erhalten werden, wie Phenol-Novolaken oder Kresol-Novolaken, auch Bisphenol-F-Novolake genannt;
- aromatischen Aminen, wie Anilin, Toluidin, 4-Aminophenol, 4,4'-Methylendiphenyldiamin, 4,4'-Methylendiphenyldi-(N-methyl)amin, 4,4'-[1,4-Phenylenbis(1-methylethyliden)]bisanilin (Bisanilin-P) oder 4,4'-[1,3-Phenylen-bis(1-methylethyliden)]bisanilin (Bisanilin-M).

Weitere geeignete Epoxidharze sind aliphatische oder cycloaliphatische Polyepoxide, insbesondere
- Glycidylether von gesättigten oder ungesättigten, verzweigten oder unverzweigten, zyklischen oder offenkettigen di-, tri- oder tetrafunktionellen C₂bis C₃₀-Alkoholen, insbesondere Ethylenglykol, Propylenglykol, Butylenglykol, Hexandiol, Octandiol, Polypropylenglykolen, Dimethylolcyclohexan, Neopentylglykol, Dibromoneopentylglykol, Rizinusöl, Trimethylolpropan, Trimethylolethan, Pentaerythrol, Sorbit oder Glycerin, oder alkoxyliertes Glycerin oder alkoxyliertes Trimethylolpropan;
- ein hydriertes Bisphenol-A-, -F- oder -A/F-Flüssigharz, beziehungsweise die Glycidylisierungsprodukte von hydriertem Bisphenol-A, -F oder -A/F;
- ein N-Glycidylderivat von Amiden oder heterocyclischen Stickstoffbasen, wie Triglycidylcyanurat oder Triglycidylisocyanurat, oder Umsetzungsprodukte von Epichlorhydrin mit Hydantoin.
- Epoxidharze aus der Oxidation von Olefinen, wie insbesondere Vinylcylohexen, Dicyclopentadien, Cyclohexadien, Cyclododecadien, Cyclododecatrien, Isopren, 1,5-Hexadien, Butadien, Polybutadien oder Divinylbenzol.

Als Epoxidharz in der Harz-Komponente bevorzugt ist ein Flüssigharz auf der Basis eines Bisphenols, insbesondere ein Diglycidylether von Bisphenol-A, Bisphenol-F oder Bisphenol-A/F, wie sie kommerziell beispielsweise von Dow, Huntsman oder Momentive erhältlich sind. Diese Flüssigharze weisen eine für Epoxidharze niedrige Viskosität und im ausgehärteten Zustand gute Eigenschaften als Beschichtung auf. Sie können Anteile von Bisphenol A-Festharz oder Bisphenol-F-Novolaken enthalten.

Die Harz-Komponente kann einen Reaktivverdünner, insbesondere einen mindestens eine Epoxidgruppe aufweisenden Reaktivverdünner, enthalten. Als Reaktivverdünner geeignet sind insbesondere die Glycidylether von ein- oder mehrwertigen Phenolen oder aliphatischen oder cycloaliphatischen Alkoholen, wie insbesondere die bereits genannten Polyglycidylether von Di- oder Polyolen, oder weiterhin Phenylglycidylether, Kresylglycidylether, Benzylglycidylether, p-n-Butylphenylglycidylether, p-tert.Butylphenylglycidylether, Nonylphenylglycidylether, Allylglycidylether, Butylglycidylether, Hexylglycidylether, 2-Ethylhexylglycidylether, oder Glycidylether von natürlichen Alkoholen wie insbesondere C₈- bis C₁₀-Alkylglycidylether oder C₁₂- bis C₁₄-Alkylglycidylether. Die Zugabe eines Reaktivverdünners zum Epoxidharz bewirkt eine Reduktion der Viskosität, und/oder eine Reduktion der Glasübergangstemperatur und/oder der mechanischen Werte.

Gegebenenfalls enthält die Epoxidharz-Zusammensetzung weitere Bestandteile, insbesondere in Epoxidharz-Zusammensetzungen üblicherweise eingesetzte Hilfs- und Zusatzstoffe, beispielsweise die Folgenden:
- Lösemittel, Verdünner, Filmbildehilfsmittel oder Extender, wie insbesondere die bereits genannten nicht einbaubaren Verdünner;
- Reaktivverdünner, insbesondere Epoxidgruppen aufweisende Reaktivverdünner, wie sie vorgängig erwähnt wurden, epoxidiertes Sojaöl oder Leinöl, Acetoacetatgruppen aufweisende Verbindungen, insbesondere acetoacetylierte Polyole, Butyrolakton, Carbonate, Aldehyde, sowie weiterhin Isocyanate oder Reaktivgruppen-aufweisende Silikone;
- Polymere, insbesondere Polyamide, Polysulfide, Polyvinylformal (PVF), Polyvinylbutyral (PVB), Polyurethane (PUR), Polymere mit Carboxylgruppen, Polyamide, Butadien-Acrylnitril-Copolymere, Styrol-Acrylnitril-Copolymere, Butadien-Styrol-Copolymere, Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere chlorsulfonierte Polyethylene oder Fluor-haltige Polymere, Sulfonamid-modifizierte Melamine oder gereinigte Montan-Wachse;
- anorganische oder organische Füllstoffe, insbesondere gemahlene oder gefällte Calciumcarbonate, welche gegebenenfalls mit Fettsäuren, insbesondere Stearaten, beschichtet sind, Baryt (Schwerspat), Talke, Quarzmehle, Quarzsand, Eisenglimmer, Dolomite, Wollastonite, Kaoline, Mica (Kalium-Aluminium-Silikat), Molekularsiebe, Aluminiumoxide, Aluminiumhydroxide, Magnesiumhydroxid, Kieselsäuren, Zemente, Gipse, Flugaschen, Russ, Graphit, Metall-Pulver wie Aluminium, Kupfer, Eisen, Zink, Silber oder Stahl, PVC-Pulver oder Hohlkugeln;
- Fasern, insbesondere Glasfasern, Kohlefasern, Metallfasern, Keramikfasern oder Kunststofffasern wie Polyamidfasern oder Polyethylenfasern;
- Pigmente, insbesondere Titandioxid und/oder Eisenoxide;
- die vorgenannten Beschleuniger;
- Rheologie-Modifizierer, insbesondere Verdicker oder Antiabsetzmittel;
- Haftverbesserer, insbesondere Organoalkoxysilane;
- Stabilisatoren gegen Oxidation, Wärme, Licht oder UV-Strahlung;
- flammhemmende Substanzen, insbesondere Aluminiumhydroxid (ATH), Magnesiumdihydroxid (MDH), Antimontrioxid, Antimonpentoxid, Borsäure (B(OH)₃), Zinkborat, Zinkphosphat, Melaminborat, Melamincyanurat, Ammoniumpolyphosphat, Melaminphosphat, Melaminpyrophosphat, polybromierte Diphenyloxide oder Diphenylether, Phosphate wie insbesondere Diphenylkresylphosphat, Resorcinol-bis(diphenylphosphat), Resorcinoldiphosphat-Oligomer, Tetraphenylresorcinoldiphosphit, Ethylendiamindiphosphat oder Bisphenol-A-bis(diphenylphosphat), Tris(chloroethyl)phosphat, Tris(chloropropyl)phosphat oder Tris(dichloroisopropyl)phosphat, Tris[3-bromo-2,2-bis-(bromomethyl)propyl]phosphat, Tetrabromo-Bisphenol-A, Bis(2,3-dibromopropylether) von Bisphenol A, bromierte Epoxidharze, Ethylen-bis(tetrabromophthalimid), Ethylen-bis(dibromonorbornandicarboximid), 1,2-Bis(tribromophenoxy)ethan, Tris(2,3-dibromopropyl)isocyanurat, Tribromophenol, Hexabromocyclododecan, Bis(hexachlorocyclopentadieno)cyclooctan oder Chlorparaffine;
- oberflächenaktive Substanzen, insbesondere Netzmittel, Verlaufsmittel, Entlüftungsmittel oder Entschäumer;
- Biozide, wie beispielsweise Algizide, Fungizide oder das Pilzwachstum hemmende Substanzen.

Bevorzugt enthält die Epoxidharz-Zusammensetzung weitere Hilfs- und Zusatzstoffe, insbesondere Netzmittel, Verlaufsmittel, Entschäumer, Stabilisatoren, Pigmente und/oder Beschleuniger, insbesondere Salicylsäure und/oder 2,4,6-Tris(dimethylaminomethyl)phenol.
Bevorzugt enthält die Epoxidharz-Zusammensetzung keinen oder nur einen geringen Gehalt an nicht einbaubaren Verdünnern, bevorzugt maximal 5 Gewichts-%, insbesondere maximal 2 Gewichts-%.

In der Epoxidharz-Zusammensetzung liegt das Verhältnis der Anzahl von gegenüber Epoxidgruppen reaktiven Gruppen gegenüber der Anzahl Epoxidgruppen bevorzugt im Bereich von 0.5 bis 1.5, insbesondere 0.7 bis 1.2.
Die in der Epoxidharz-Zusammensetzung vorhandenen Aminwasserstoffe und gegebenenfalls vorhandene weitere gegenüber Epoxidgruppen reaktive Gruppen reagieren mit den Epoxidgruppen unter deren Ringöffnung (Additionsreaktion). Als Ergebnis dieser Reaktionen polymerisiert die Zusammensetzung und härtet schliesslich aus. Dem Fachmann ist bekannt, dass primäre Aminogruppen gegenüber Epoxidgruppen difunktionell sind und eine primäre Aminogruppe somit als zwei gegenüber Epoxidgruppen reaktive Gruppen zählt.

Die beiden Komponenten der Epoxidharz-Zusammensetzung werden jeweils in einem eigenen Gebinde gelagert. Weitere Bestandteile der Epoxidharz-Zusammensetzung können als Bestandteil der Harz- oder der Härter-Komponente vorhanden sein, wobei gegenüber Epoxidgruppen reaktive weitere Bestandteile bevorzugt ein Bestandteil der Härter-Komponente sind. Ein geeignetes Gebinde zum Lagern der Harz- oder der Härter-Komponente ist insbesondere ein Fass, ein Hobbock, ein Beutel, ein Eimer, eine Büchse, eine Kartusche oder eine Tube. Die Komponenten sind lagerfähig, das heisst, dass sie vor ihrer Anwendung während mehreren Monaten bis zu einem Jahr und länger aufbewahrt werden können, ohne dass sie sich in ihren jeweiligen Eigenschaften in einem für ihren Gebrauch relevanten Ausmass verändern. Zur Anwendung der Epoxidharz-Zusammensetzung werden die Harz- und die Härter-Komponente kurz vor oder während der Applikation miteinander vermischt. Das Mischungsverhältnis zwischen den beiden Komponenten wird bevorzugt so gewählt, dass die gegenüber Epoxidgruppen reaktiven Gruppen der Härter-Komponente in einem geeigneten Verhältnis zu den Epoxidgruppen der Harz-Komponente stehen, wie vorgängig beschrieben. In Gewichtsteilen liegt das Mischungsverhältnis zwischen der Harz-Komponente und der Härter-Komponente üblicherweise im Bereich von 1:10 bis 10:1.
Die Vermischung der beiden Komponenten erfolgt mittels eines geeigneten Verfahrens; sie kann kontinuierlich oder batchweise erfolgen. Falls das Vermischen vor der Applikation erfolgt, muss darauf geachtet werden, dass zwischen dem Vermischen der Komponenten und der Applikation nicht zu viel Zeit vergeht, da es dadurch zu Störungen, wie beispielsweise einem verlangsamten oder unvollständigen Aufbau der Haftung zum Substrat, kommen kann. Die Vermischung erfolgt insbesondere bei Umgebungstemperatur, welche typischerweise im Bereich von etwa 5 bis 50°C, bevorzugt bei etwa 10 bis 30°C, liegt.
Mit der Vermischung der beiden Komponenten beginnt die Aushärtung durch chemische Reaktion, wie vorgängig beschrieben. Die Aushärtung erfolgt typischerweise bei einer Temperatur im Bereich von 0 bis 150 °C. Bevorzugt erfolgt sie bei Umgebungstemperatur und erstreckt sich typischerweise über einige Tage bis Wochen. Die Dauer hängt unter anderem von der Temperatur, der Reaktivität der Bestandteile und deren Stöchiometrie sowie der Gegenwart von Beschleunigern ab.

Die Applikation der Epoxidharz-Zusammensetzung erfolgt auf mindestens ein Substrat, wobei die Folgenden besonders geeignet sind:
- Glas, Glaskeramik, Beton, Mörtel, Backstein, Ziegel, Gips oder Natursteine wie Granit oder Marmor;
- Metalle oder Legierungen wie Aluminium, Eisen, Stahl oder Buntmetalle, oder oberflächenveredelte Metalle oder Legierungen wie verzinkte oder verchromte Metalle;
- Leder, Textilien, Papier, Holz, mit Harzen, beispielsweise Phenol-, Melamin- oder Epoxidharzen, gebundene Holzwerkstoffe, Harz-Textil-Verbundwerkstoffe oder weitere sogenannte Polymer-Composites;
- Kunststoffe, insbesondere Hart- oder Weich-PVC, ABS, Polycarbonat (PC), Polyamid (PA), Polyester, PMMA, Epoxidharze, PUR, POM, PO, PE, PP, EPM oder EPDM, wobei die Kunststoffe gegebenenfalls mittels Plasma, Corona oder Flammen oberflächenbehandelt sind;
- Faserverstärkte Kunststoffe, wie Kohlefaser-verstärkte Kunststoffe (CFK), Glasfaser-verstärkte Kunststoffe (GFK) oder Sheet Moulding Compounds (SMC);
- beschichtete Substrate, wie pulverbeschichtete Metalle oder Legierungen;
- Farben oder Lacke.
Die Substrate können bei Bedarf vor dem Applizieren der Epoxidharz-Zusammensetzung vorbehandelt werden. Derartige Vorbehandlungen umfassen insbesondere physikalische und/oder chemische Reinigungsverfahren, beispielsweise Schleifen, Sandstrahlen, Kugelstrahlen, Bürsten und/oder Abblasen, sowie weiterhin Behandeln mit Reinigern oder Lösemitteln oder das Aufbringen eines Haftvermittlers, einer Haftvermittlerlösung oder eines Primers.

Die beschriebene Epoxidharz-Zusammensetzung ist vorteilhaft verwendbar als Faserverbundmatrix für Faserverbundwerkstoffe (Composites), als Vergussmasse, Dichtstoff, Klebstoff, Belag, Beschichtung, Anstrich, Lack, Versiegelung, Grundierung oder Primer, sowie für zementäre Produkte wie Reparaturmörtel oder Vergussmörtel (Grout) auf Zementbasis.

Bevorzugt verwendbar ist sie als
- Faserverbundmatrix für Faserverbundwerkstoffe (Composites) wie insbesondere CFK oder GFK;
- Klebstoff, insbesondere als Karrosserieklebstoff, Sandwichelementklebstoff, Halbschalenklebstoff für Rotorblätter von Windkraftanlagen, Brückenelementklebstoff oder Verankerungsklebstoff; oder als
- Beschichtung, Belag oder Anstrich, insbesondere als Lack, Versiegelung, Grundierung oder Primer für Bau- oder Industrieanwendungen, oder als Bodenbelag oder Bodenbeschichtung für Innenräume wie Büros, Industriehallen, Turnhallen oder Kühlräume, oder als Bodenbelag oder Bodenbeschichtung im Aussenbereich für Balkone, Terrassen, Parkdecks, Brücken oder Dächer, oder als Schutzbeschichtung für Beton, Zement, Metalle, Kunststoffe oder Holz, beispielsweise zur Oberflächenversiegelung von Holzkonstruktionen, Fahrzeugen, Ladeflächen, Tanks, Silos, Schächten, Rohrleitungen, Pipelines, Maschinen oder Stahlkonstruktionen, beispielsweise von Schiffen, Piers, Offshore-Plattformen, Schleusentoren, Wasserkraftwerken, Flussbauten, Schwimmbädern, Windkraftanlagen, Brücken, Kaminen, Kranen oder Spundwänden, insbesondere auch für schweren Korrosionsschutz; oder als Voranstrich, Haftanstrich, Korrosionsschutz-Primer oder zur Hydrophobierung von Oberflächen.

Insbesondere verwendbar ist die beschriebene Epoxidharz-Zusammensetzung in emissionsarmen Produkten mit Öko-Gütesiegeln, beispielsweise nach Emicode (EC1 Plus), AgBB, DIBt, Der Blaue Engel, AFSSET, RTS (M1) und US Green Building Council (LEED).

Auf die vollständig oder teilweise ausgehärtete Epoxidharz-Zusammensetzung kann insbesondere bei ihrer Verwendung als Beschichtung, Belag oder Anstrich eine weitere Beschichtung, ein weiterer Belag, oder ein weiterer Anstrich appliziert werden, wobei es sich bei dieser weiteren Schicht ebenfalls um eine Epoxidharz-Zusammensetzung handeln kann, aber auch um ein anderes Material, insbesondere um eine Polyurethan- oder Polyharnstoff-Beschichtung.

Als Beschichtung wird die Epoxidharz-Zusammensetzung vorteilhaft in einem Verfahren zum Beschichten verwendet, wobei sie eine flüssige Konsistenz mit niedriger Viskosität und guten Verlaufseigenschaften aufweist und insbesondere als selbstverlaufende oder thixotropierte Beschichtung auf überwiegend ebene Flächen oder als Anstrich appliziert wird. Bevorzugt weist die Epoxidharz-Zusammensetzung bei dieser Applikation unmittelbar nach dem Vermischen der Harz- und der Härter-Komponente eine Viskosität, gemessen bei 20°C, im Bereich von 300 bis 4'000 mPa·s, bevorzugt im Bereich von 300 bis 3'000 mPa·s, auf. Die vermischte Zusammensetzung wird innerhalb der Verarbeitungszeit flächig als dünner Film mit einer Schichtdicke von typischerweise etwa 50 µm bis etwa 5 mm auf ein Substrat appliziert, typischerweise bei Umgebungstemperatur. Die Applikation erfolgt insbesondere durch Aufgiessen auf das zu beschichtende Substrat und anschliessendem gleichmässigem Verteilen mit Hilfe beispielsweise eines Rakels oder einer Zahntraufel. Die Applikation kann aber auch mit einem Pinsel oder Roller oder als Spritzapplikation erfolgen, beispielsweise als Korrosionsschutzbeschichtung auf Stahl.
Bei der Aushärtung entstehen typischerweise weitgehend klare, glänzende und nichtklebrige Filme von hoher Härte, welche eine gute Haftung zu verschiedensten Substraten aufweisen.

Aus der Anwendung der Epoxidharz-Zusammensetzung entsteht ein Artikel umfassend die ausgehärtete Zusammensetzung aus der Aushärtung der beschriebenen Epoxidharz-Zusammensetzung.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

"AHEW" steht für das Aminwasserstoff-Equivalentgewicht.

"EEW" steht für das Epoxid-Equivalentgewicht.

Als "Normklima" wird eine Temperatur von 23±1 °C und eine relative Luftfeuchtigkeit von 50±5% bezeichnet. "NK" steht für "Normklima".

### Beschreibung der Messmethoden:

**Infrarotspektren** (FT-IR) wurden als unverdünnte Filme auf einem mit horizontaler ATR-Messeinheit mit ZnSe-Kristall ausgestatteten FT-IR Gerät 1600 von Perkin-Elmer gemessen; die Absorptionsbanden sind in Wellenzahlen (cm⁻¹) angegeben (Messfenster: 4000-650 cm⁻¹).

Die **Viskosität** wurde auf einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 s⁻¹) gemessen.

Die **Aminzahl** wurde durch Titration bestimmt (mit 0.1N HClO₄ in Essigsäure gegen Kristallviolett).

### Verwendete Substanzen:

| | |
|---|---|
| Araldite® GY 250: | Bisphenol-A-Diglycidylether, EEW ca. 187.5 g/Eq (von Huntsman) |
| Araldite® DY-E: | Monoglycidylether von C₁₂- bis C₁₄-Alkoholen, EEW ca. 290 g/Eq (von Huntsman) |
| Ancamine® K 54: | 2,4,6-Tris(dimethylaminomethyl)phenol (von Air Products) |
| EP-Addukt 1: | Umsetzungsprodukt aus 116.0 g 1,5-Diamino-2-methylpentan mit 200.2 g Araldite® DY-K; AHEW ca. 109.5 g/Eq; Viskosität (20°C) ca. 13.1 Pa·s |
| Araldite® DY-K: | Kresylglycidylether, EEW ca. 182 g/Eq (von Huntsman) |
| Jeffamine® D-230: | Polyoxypropylendiamin mit mittlerem Molekulargewicht ca. 240 g/mol, AHEW ca. 60 g/Eq (von Huntsman) |
| Gaskamine® 240: | Styrolisiertes 1,3-Bis(aminomethyl)benzol; AHEW 103 g/Eq; Viskosität (20°C) 165 mPa·s (von Mitsubishi Gas Chemical) |
| N-Benzyl-1,2-propandiamin: | Reaktionsmischung, hergestellt wie im Folgenden beschrieben |

### N-Benzyl-1,2-propandiamin:

In einem Rundkolben wurden 444.8 g (6 mol) 1,2-Propandiamin unter Stickstoffatmosphäre bei Raumtemperatur vorgelegt. Unter gutem Rühren wurde langsam eine Lösung aus 212.2 g (2 mol) Benzaldehyd in 1'500 ml Isopropanol dazugetropft und 2 Stunden nachgerührt. Die Reaktionsmischung wurde anschliessend bei einem Wasserstoff-Druck von 90 bar, einer Temperatur von 85 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65 °C eingeengt, wobei unreagiertes 1,2-Propandiamin und Isopropanol entfernt wurden. Es wurde eine klare, leicht gelbliche Flüssigkeit erhalten. Davon wurden 300 g bei 80 °C unter Vakuum destilliert, wobei 237.5 g Destillat bei einer Dampftemperatur von 60 bis 63 °C und 0.08 bis 0.09 bar aufgefangen wurden. Erhalten wurde eine farblose Flüssigkeit mit einer Viskosität von 8.5 mPa·s bei 20 °C und einer Aminzahl von 682 mg KOH/g, welche gemäss ¹H-NMR eine Mischung aus N¹-Benzyl-1,2-propandiamin und N²-Benzyl-1,2-propandiamin im Verhältnis von ca. 2/1 enthielt.

### Herstellung von Aminen der Formel (I)

### Amin 1: Reaktionsgemisch enthaltend 1,4-Bis(2-aminopropylaminomethyl)-benzol

In einem Rundkolben wurden 74.1 g (1.0 mol) 1,2-Propylendiamin unter Stickstoffatmosphäre bei Raumtemperatur vorgelegt. Unter gutem Rühren wurde langsam eine Lösung von 26.8 g (0.2 mol) Terephthalaldehyd in 500 ml Dioxan dazugetropft und 30 min. nachgerührt. Die Reaktionsmischung wurde bei einem Wasserstoff-Druck von 85 bar, einer Temperatur von 85 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurden am Rotationsverdampfer bei 65 °C und 1 mbar das überschüssige 1,2-Propylendiamin und das Lösemittel entfernt. Erhalten wurde eine klare gelbliche Flüssigkeit mit einer Viskosität von 4.13 Pa·s bei 20 °C und einer Aminzahl von 768.8 mg KOH/g.
FT-IR: 2959, 2913, 2890, 2852, 1454, 1366, 1288, 1254, 1117, 1082, 1048, 890, 870.

### Amin 2: Reaktionsgemisch enthaltend 1,4-Bis(2-aminopropylaminomethyl)-benzol

In einem Rundkolben wurden 111.2 g (1.5 mol) 1,2-Propylendiamin unter Stickstoffatmosphäre bei Raumtemperatur vorgelegt. Unter gutem Rühren wurde langsam eine auf 50 °C aufgewärmte Lösung von 26.8 g (0.2 mol) Terephthalaldehyd in 600 ml Dioxan dazugetropft und 30 min. nachgerührt. Die Reaktionsmischung wurde bei einem Wasserstoff-Druck von 90 bar, einer Temperatur von 90 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurden am Rotationsverdampfer bei 65 °C und 1 mbar das überschüssige 1,2-Propylendiamin und das Lösemittel entfernt. Erhalten wurde eine klare hellgelbe Flüssigkeit mit einer Viskosität von 0.24 Pa·s bei 20 °C und einer Aminzahl von 849.2 mg KOH/g.
FT-IR: 2959, 2800, 1567, 1500, 1440, 1367, 1100, 1010, 1048, 810.

### Herstellung von Härtern und Epoxidharz-Zusammensetzungen

Für jedes Beispiel wurden die in der Tabelle 1 oder 2 angegebenen Inhaltsstoffe in den angegebenen Mengen (in Gewichtsteilen) der Härter-Komponente mittels eines Zentrifugalmischers (SpeedMixer™ DAC 150, FlackTek Inc.) vermischt und unter Ausschluss von Feuchtigkeit aufbewahrt.

Ebenso wurden die in der Tabelle 1 oder 2 angegebenen Inhaltsstoffe der Harz-Komponente verarbeitet und aufbewahrt.

Anschliessend wurden die beiden Komponenten jeder Zusammensetzung mittels des Zentrifugalmischers zu einer homogenen Flüssigkeit verarbeitet und diese unverzüglich folgendermassen geprüft:
10 Minuten nach dem Vermischen wurde die Viskosität bei 20°C bestimmt (**"Viskosität (10')**").

Ein erster Film wurde in einer Schichtdicke von 500 µm auf eine Glasplatte aufgezogen und dieser im Normklima gelagert bzw. ausgehärtet. An diesem Film wurde die **Königshärte** (Pendelhärte nach König, gemessen nach DIN EN ISO 1522) nach 1 Tag ("Königshärte (1d NK)"), nach 2 Tagen ("Königshärte (2d NK)"), nach 4 Tagen ("Königshärte (4d NK)"), nach 7 Tagen ("Königshärte (7d NK)") und nach 14 Tagen ("Königshärte (14d NK)") bestimmt. Nach 14 Tagen wurde der Aspekt des Films beurteilt (in der Tabelle mit "Aspekt (NK)" bezeichnet). Als "schön" wurde dabei ein Film bezeichnet, welcher klar war und eine glänzende und nichtklebrige Oberfläche ohne Struktur aufwies. Als "Struktur" wird dabei jegliche Art von Zeichnung oder Muster auf der Oberfläche bezeichnet.

Ein zweiter Film wurde in einer Schichtdicke von 500 µm auf eine Glasplatte aufgezogen und dieser unmittelbar nach dem Applizieren während 7 Tagen bei 8 °C und 80 % relativer Feuchtigkeit und anschliessend während 3 Wochen im NK gelagert, beziehungsweise ausgehärtet. 24 Stunden nach der Applikation wurde ein Flaschendeckel aus Polypropylen auf den Film aufgesetzt, unter welchem ein feuchtes Schwämmchen platziert war. Nach weiteren 24 Stunden wurde das Schwämmchen und der Deckel entfernt und an einer neuen Stelle des Films platziert, wo es nach 24 Stunden wieder entfernt und neu platziert wurde, insgesamt 4 mal. Anschliessend wurde der Aspekt dieses Films beurteilt (in den Tabellen mit "Aspekt (8°/80%)" bezeichnet), auf die gleiche Weise wie für den Aspekt (NK) beschrieben. Dabei wurde jeweils auch die Anzahl Markierungen angegeben, die im Film durch das feuchte Schwämmchen und/ oder den aufgesetzten Deckel sichtbar waren. An den so ausgehärteten Filmen wurde wiederum die Königshärte bestimmt, jeweils nach 7 Tagen bei 8 °C und 80 % relativer Feuchtigkeit ("Königshärte (7d 8°/80%)"), dann nach weiteren 2 Tagen im Normklima ("Königshärte (+2d NK)"), 7 Tagen im Normklima ("Königshärte (+7d NK)") und nach 2 Wochen im Normklima ("Königshärte (+14d NK)").

Die Resultate sind in der Tabelle 1 oder 2 angegeben.

Bei den Epoxidharz-Zusammensetzungen **EZ-1** bis **EZ-8** handelt es sich um erfindungsgemässe Beispiele. Bei den Epoxidharz-Zusammensetzungen ***Ref-1*** und ***Ref-2*** handelt es sich um Vergleichsbeispiele.

**Tabelle 1: Zusammensetzung und Eigenschaften von EZ-1 bis EZ-4 und Ref-1 bis Ref-2. "n.b." steht für "nicht bestimmt"**

| **Beispiel** | | | ***EZ-1*** | ***EZ-2*** | ***EZ-3*** | ***EZ-4*** | ***Ref-1*** | ***Ref-2*** |
|---|---|---|---|---|---|---|---|---|
| **Harz-Komp.:** | | | | | | | | |
| | Araldite® GY-250 | | 167.2 | 167.2 | 167.2 | 167.2 | 167.2 | 167.2 |
| | Araldite® DY-E | | 31.8 | 31.8 | 31.8 | 31.8 | 31.8 | 31.8 |

| **Härter-Komp.:** | | | | | | | | |
|---|---|---|---|---|---|---|---|---|
| | Amin 1 | | 39.4 | 41.7 | 54.3 | - | - | - |
| | Amin 2 | | - | - | - | 41.7 | - | - |
| | EP-Addukt 1 | | - | - | - | - | 109.5 | 109.5 |
| | Ancamine® K 54 | | 4.8 | - | - | - | 6.2 | - |
| Viskosität (10') [Pa·s] | | | 2.4 | 2.3 | 2.6 | 1.6 | 4.6 | 4.6 |
| Königshärte [s] | | (1d NK) | 91 | 39 | 109 | 109 | 108 | 63 |
| | | (2d NK) | 134 | 71 | 140 | 140 | 147 | 85 |
| | | (4d NK) | 167 | 94 | 155 | 144 | 168 | 105 |
| | | (7d NK) | 188 | 104 | 157 | 161 | 176 | 121 |
| | | (14d NK) | 203 | 111 | 161 | n.b. | 185 | 140 |
| Aspekt (NK) | | | schön | schön | schön | schön | schön | schön |
| Königshärte [s] | | (7d 8°/80%) | 42 | 21 | 46 | 11 | 53 | 37 |
| | | (+2d NK) | 59 | 28 | 64 | 15 | 129 | 94 |
| | | (+7d NK) | 76 | 34 | 77 | n.b. | 161 | 113 |
| | | (+14d NK) | 125 | 34 | 80 | n.b. | 183 | 125 |
| Aspekt (8°/80%) | | | leicht matt | leicht matt | leicht matt | schön | leicht matt | leicht matt |
| Anzahl Markierungen | | | 1 | 1 | 1 | keine | 2 | 2 |

**Tabelle 2: Zusammensetzung und Eigenschaften von EZ-5 bis EZ-8. "n.b." steht für "nicht bestimmt"**

| **Beispiel** | | | ***EZ-5*** | ***EZ-6*** | ***EZ-7*** | ***EZ-8*** |
|---|---|---|---|---|---|---|
| **Harz-Komp.:** | | | | | | |
| | Araldite® GY-250 | | 167.2 | 167.2 | 167.2 | 167.2 |
| | Araldite® DY-E | | 31.8 | 31.8 | 31.8 | 31.8 |

| **Härter-Komp.:** | | | | | | |
|---|---|---|---|---|---|---|
| | Amin 1 | | 29.2 | 20.9 | 20.9 | - |
| | Amin 2 | | - | - | - | 25.0 |
| | Jeffamine® D-230 | | 18.0 | - | - | - |
| | Gaskamine® 240 | | - | 51.5 | - | - |
| | N-Benzyl-1,2-propandiamin | | - | - | 27.4 | 27.4 |
| Viskosität (10') [Pa·s] | | | 1.11 | 0.93 | 0.71 | 0.58 |
| Königshärte [s] | | (1d NK) | 15 | 55 | 50 | 109 |
| | | (2d NK) | 80 | 123 | 136 | 155 |
| | | (4d NK) | 97 | 144 | 153 | 176 |
| | | (7d NK) | 106 | 157 | 158 | 185 |
| | | (14d NK) | 132 | 178 | 170 | n.b. |
| Aspekt (NK) | | | schön | schön | schön | schön |
| Königshärte [s] | | (7d 8°/80%) | 3 | 24 | 25 | 38 |
| | | (+2d NK) | 4 | 50 | 40 | 50 |
| | | (+7d NK) | 6 | 66 | 55 | n.b. |
| | | (+14d NK) | 8 | 85 | 55 | n.b. |
| Aspekt (8°/80%) | | | klebrig | leicht matt | leicht matt | schön |
| Anzahl Markierungen | | | 1 | 1 | 1 | keine |

## Patentansprüche

1. Amin der Formel (I), 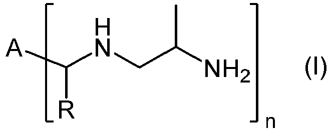 wobei
n für 2 oder 3 steht,
R für einen Wasserstoff-Rest oder für Methyl oder Phenyl steht, und
A für einen, gegebenenfalls Sauerstoff- oder Schwefel- oder Stickstoffatome aufweisenden, n-wertigen Kohlenwasserstoff-Rest mit 5 bis 20 C-Atomen, welcher mindestens einen cycloaliphatischen oder aromatischen Ring enthält, steht.

2. Amin der Formel (I) gemäss Anspruch 1, **dadurch gekennzeichnet, dass** n für 2 steht.

3. Amin der Formel (I) gemäss einem der Ansprüche 1 oder 2, **dadurch gekennzeichnet, dass** R für einen Wasserstoff-Rest steht.

4. Amin der Formel (I) gemäss einem der Ansprüche 1 bis 3, **dadurch gekennzeichnet, dass** A für einen gegebenenfalls substituierten Phenylen- oder Cyclohexylen- oder Dicycloheptylen- oder Tricyclodecylen- oder Pentacyclopentadecylen- oder Furandiyl- oder Tetrahydrofurandiyl- oder Thiophendiyl- oder Tetrahydrothiophendiyl- oder N,N'-Piperazin-bis(2,2-dimethylpropan)diyl-Rest steht.

5. Amin der Formel (I) gemäss einem der Ansprüche 1 bis 4, **dadurch gekennzeichnet, dass** es 1,4-Bis(2-aminopropylaminomethyl)benzol oder 1,4-Bis(2-aminopropylaminomethyl)cyclohexan ist.

6. Verfahren zur Herstellung eines Amins der Formel (I) gemäss einem der Ansprüche 1 bis 5, dadurch gekennzeichent, dass 1,2-Propylendiamin mit mindestens einer di- oder trifunktionellen Carbonylverbindung der Formel (III) und Wasserstoff reduktiv alkyliert wird,
wobei n für 2 oder 3 steht,
R für einen Wasserstoff-Rest oder für Methyl oder Phenyl steht, und
A für einen, gegebenenfalls Sauerstoff- oder Schwefel- oder Stickstoffatome aufweisenden, n-wertigen Kohlenwasserstoff-Rest mit 5 bis 20 C-Atomen, welcher mindestens einen cycloaliphatischen oder aromatischen Ring enthält, steht.

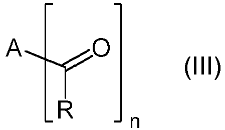

7. Verfahren gemäss Anspruch 6, **dadurch gekennzeichnet, dass** 1,2-Propylendiamin gegenüber den Carbonylgruppen der Carbonylverbindung der Formel (III) im stöchiometrischen Überschuss eingesetzt wird.

8. Verwendung eines Amins der Formel (I) gemäss einem der Ansprüche 1 bis 5 als Härter für Epoxidharze.

9. Härter für Epoxidharze enthaltend mindestens ein Amin der Formel (I) gemäss einem der Ansprüche 1 bis 5 und mindestens ein weiteres Amin und/oder mindestens einen Beschleuniger.

10. Härter gemäss Anspruch 9, **dadurch gekennzeichnet, dass** der Beschleuniger Salicylsäure oder 2,4,6-Tris(dimethylaminomethyl)phenol oder eine Kombination davon ist.

11. Härter gemäss einem der Ansprüche 9 oder 10, **dadurch gekennzeichnet, dass** das weitere Amin 1-(2-Aminopropylaminomethyl)-4-(1-aminoprop-2-ylaminomethyl)benzol oder 1,4-Bis(1-aminoprop-2-ylaminomethyl)benzol oder 1-(2-Aminopropylaminomethyl)-4-(1-aminoprop-2-ylaminomethyl)cyclohexan oder 1,4-Bis(1-aminoprop-2-ylaminomethyl)cyclohexan umfasst.

12. Härter gemäss einem der Ansprüche 9 bis 11, **dadurch gekennzeichnet, dass** das weitere Amin ein partiell styrolisiertes Polyamin oder ein Produkt aus der reduktiven Alkylierung von primären aliphatischen Polyaminen mit monofunktionellen Aldehyden oder Ketonen umfasst.

13. Härter gemäss einem der Ansprüche 9 bis 12, **dadurch gekennzeichnet, dass** er maximal 10 Gewichts-% nicht einbaubare Verdünner enthält.

14. Epoxidharz-Zusammensetzung umfassend
- eine Harz-Komponente enthaltend mindestens ein Epoxidharz und
- eine Härter-Komponente enthaltend mindestens ein Amin der Formel (I) gemäss einem der Ansprüche 1 bis 5.

15. Verwendung einer Epoxidharz-Zusammensetzung gemäss Anspruch 14, als Faserverbundmatrix für Faserverbundwerkstoffe oder als Klebstoff oder als Beschichtung, Belag oder Anstrich.

## Claims

1. Amine of the formula (I) 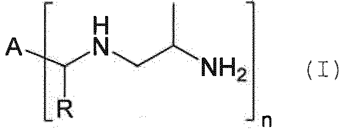 where
n is 2 or 3,
R is a hydrogen radical or is methyl or phenyl, and
A is an n-valent hydrocarbon radical having 5 to 20 C atoms which optionally contains oxygen or sulfur or nitrogen atoms and which comprises at least one cycloaliphatic or aromatic ring.

2. Amine of the formula (I) according to Claim 1, **characterized in that** n is 2.

3. Amine of the formula (I) according to either of Claims 1 and 2, **characterized in that** R is a hydrogen radical.

4. Amine of the formula (I) according to any of Claims 1 to 3, **characterized in that** A is an optionally substituted phenylene or cyclohexylene or dicycloheptylene or tricyclodecylene or pentacyclopentadecylene or furandiyl or tetrahydrofurandiyl or thiophenediyl or tetrahydrothiophenediyl or N,N'-piperazine-bis(2,2-dimethylpropane)diyl radical.

5. Amine of the formula (I) according to any of Claims 1 to 4, **characterized in that** it is 1,4-bis(2-aminopropylaminomethyl)benzene or 1,4-bis(2-aminopropylaminomethyl)cyclohexane.

6. Process for preparing an amine of the formula (I) according to any of Claims 1 to 5, **characterized in that** 1,2-propylenediamine is subjected to reductive alkylation with at least one di- or trifunctional carbonyl compound of the formula (III) and hydrogen, where n is 2 or 3,
R is a hydrogen radical or is methyl or phenyl, and
A is an n-valent hydrocarbon radical having 5 to 20 C atoms which optionally contains oxygen or sulfur or nitrogen atoms and which comprises at least one cycloaliphatic or aromatic ring.

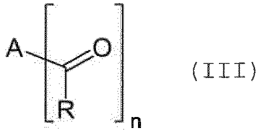

7. Process according to Claim 6, **characterized in that** 1,2-propylenediamine is used in stoichiometric excess over the carbonyl groups of the carbonyl compound of the formula (III).

8. Use of an amine of the formula (I) according to any of Claims 1 to 5 as hardener for epoxy resins.

9. Hardener for epoxy resins, comprising at least one amine of the formula (I) according to any of Claims 1 to 5 and at least one further amine and/or at least one accelerator.

10. Hardener according to Claim 9, **characterized in that** the accelerator is salicylic acid or 2,4,6-tris(dimethylaminomethyl)phenol or a combination thereof.

11. Hardener according to either of Claims 9 and 10, **characterized in that** the further amine comprises 1-(2-aminopropylaminomethyl)-4-(1-aminoprop-2-ylaminomethyl)benzene or 1,4-bis(1-aminoprop-2-ylaminomethyl)benzene or 1-(2-aminopropylaminomethyl)-4-(1-aminoprop-2-ylaminomethyl)cyclohexane or 1,4-bis(1-aminoprop-2-ylaminomethyl)cyclohexane.

12. Hardener according to any of Claims 9 to 11, **characterized in that** the further amine comprises a partially styrenized polyamine or a product of the reductive alkylation of primary aliphatic polyamines with monofunctional aldehydes or ketones.

13. Hardener according to any of Claims 9 to 12, **characterized in that** it contains not more than 10 weight% of unincorporable diluents.

14. Epoxy resin composition comprising
- a resin component comprising at least one epoxy resin and
- a hardener component comprising at least one amine of the formula (I) according to any of Claims 1 to 5.

15. Use of an epoxy resin composition according to Claim 14 as fiber composite matrix for fiber composite materials or as adhesive or as coating, covering or finish.

## Revendications

1. Amine de formule (I) 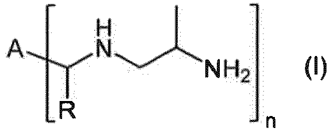 dans laquelle
n représente 2 ou 3,
R représente un radical hydrogène ou méthyle ou phényle, et
A représente un radical hydrocarboné n-valent de 5 à 20 atomes C comprenant éventuellement des atomes d'oxygène ou de soufre ou d'azote, qui contient au moins un cycle cycloaliphatique ou aromatique.

2. Amine de formule (I) selon la revendication 1, **caractérisée en ce que** n représente 2.

3. Amine de formule (I) selon l'une quelconque des revendications 1 ou 2, **caractérisée en ce que** R représente un radical hydrogène.

4. Amine de formule (I) selon l'une quelconque des revendications 1 à 3, **caractérisée en ce qu'**A représente un radical phénylène ou cyclohexylène ou dicycloheptylène ou tricyclodécylène ou pentacyclopentadécylène ou furane-diyle ou tétrahydrofurane-diyle ou thiophène-diyle ou tétrahydrothiophène-diyle ou N,N'-pipérazine-bis(2,2-diméthylpropane)diyle éventuellement substitué.

5. Amine de formule (I) selon l'une quelconque des revendications 1 à 4, **caractérisée en ce qu'**elle consiste en le 1,4-bis(2-aminopropylaminométhyl)benzène ou le 1,4-bis(2-aminopropylaminométhyl)cyclohexane.

6. Procédé de fabrication d'une amine de formule (I) selon l'une quelconque des revendications 1 à 5, **caractérisé en ce que** de la 1,2-propylène-diamine est soumise à une alkylation réductrice avec au moins un composé de carbonyle di- ou trifonctionnel de formule (III) et de l'hydrogène, dans laquelle
n représente 2 ou 3,
R représente un radical hydrogène ou méthyle ou phényle, et
A représente un radical hydrocarboné n-valent de 5 à 20 atomes C comprenant éventuellement des atomes d'oxygène ou de soufre ou d'azote, qui contient au moins un cycle cycloaliphatique ou aromatique

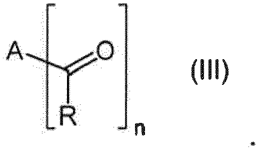

7. Procédé selon la revendication 6, **caractérisé en ce que** la 1,2-propylène-diamine est utilisée en excès stoechiométrique par rapport aux groupes carbonyle du composé de carbonyle de formule (III).

8. Utilisation d'une amine de formule (I) selon l'une quelconque des revendications 1 à 5 en tant que durcisseur pour résines époxydes.

9. Durcisseur pour résines époxydes contenant au moins une amine de formule (I) selon l'une quelconque des revendications 1 à 5 et au moins une autre amine et/ou au moins un accélérateur.

10. Durcisseur selon la revendication 9, **caractérisé en ce que** l'accélérateur est l'acide salicylique ou le 2,4,6-tris(diméthylaminométhyl)phénol ou une combinaison de ceux-ci.

11. Durcisseur selon l'une quelconque des revendications 9 ou 10, **caractérisé en ce que** l'autre amine comprend le 1-(2-aminopropylaminométhyl)-4-(1-aminoprop-2-ylaminométhyl)benzène ou le 1,4-bis(1-aminoprop-2-ylaminométhyl)benzène ou le 1-(2-aminopropylaminométhyl)-4-(1-aminoprop-2-ylaminométhyl)cyclohexane ou le 1,4-bis(1-aminoprop-2-ylaminométhyl)cyclohexane.

12. Durcisseur selon l'une quelconque des revendications 9 à 11, **caractérisé en ce que** l'autre amine comprend une polyamine partiellement styrénisée ou un produit de l'alkylation réductrice de polyamines aliphatiques primaires avec des aldéhydes ou des cétones monofonctionnels.

13. Durcisseur selon l'une quelconque des revendications 9 à 12, **caractérisé en ce qu'**il contient au plus 10 % en poids de diluants non incorporables.

14. Composition de résine époxyde, comprenant :
- un composant résine contenant au moins une résine époxyde et
- un composant durcisseur contenant au moins une amine de formule (I) selon l'une quelconque des revendications 1 à 5.

15. Utilisation d'une composition de résine époxyde selon la revendication 14 en tant que matrice composite fibreuse pour matériaux composites fibreux ou en tant qu'adhésif ou en tant que revêtement, garniture ou enduit.
